# EUROPEAN PATENT SPECIFICATION

(11) **EP 1 844 721 B1**
(45) Date of publication and mention of the grant of the patent: **02.12.2009**
(21) Application number: 07007259.0
(22) Date of filing: 05.04.2007
(51) Int. Cl.: A61B 17/64, A61B 17/66

(54) **All-purpose external fixator for orthopedic uses**
Externer Allzweck-Fixierer für orthopädische Verwendungen
Fixateur externe polyvalent pour utilisations orthopédiques

(30) Priority: 05.04.2006 IT VI20060101
(43) Date of publication of application: 17.10.2007
(73) Proprietor: Permedica S.p.A., 23807 Merate (Lecco) (IT); Castaman, Enrico, 36100 Vicenza (IT)
(72) Inventor: Sinico, Claudio, 36010 Monticello Conte Otto (VI) (IT); Castaman, Enrico, 36100 Vicenza (IT)
(74) Representative: Bonini, Ercole

(56) References cited:
- EP-A- 0 880 942
- WO-A-88/05287
- CH-A5- 638 390
- US-A- 5 803 924

## Description

This invention relates to an all-purpose external fixator particularly suitable for use in the field of orthopedic traumatology.

It is well known that external fixation of the bone stumps of a fractured limb or other part of the human skeleton is widely used in the field of orthopedic traumatology because it enables a correct juxtaposition of the stumps to enable the correct reconstruction of the anatomy of the segment.

Moreover, external fixation does not interfere with the aeration of the limb or other area being treated and thus avoids the decline in muscle tone typically encountered when plaster casts are used.

Finally, external fixation with fixators that comprise a shock-absorbing deflection unit to enable the elastic deflection of the implant facilitates the optimal formation of the bone callus in the area affected by the fracture.

Various known types of all-purpose external fixator are used singly, in pairs, or in combination with arched connection elements respectively to create monolateral, bilateral or three-dimensional implants.

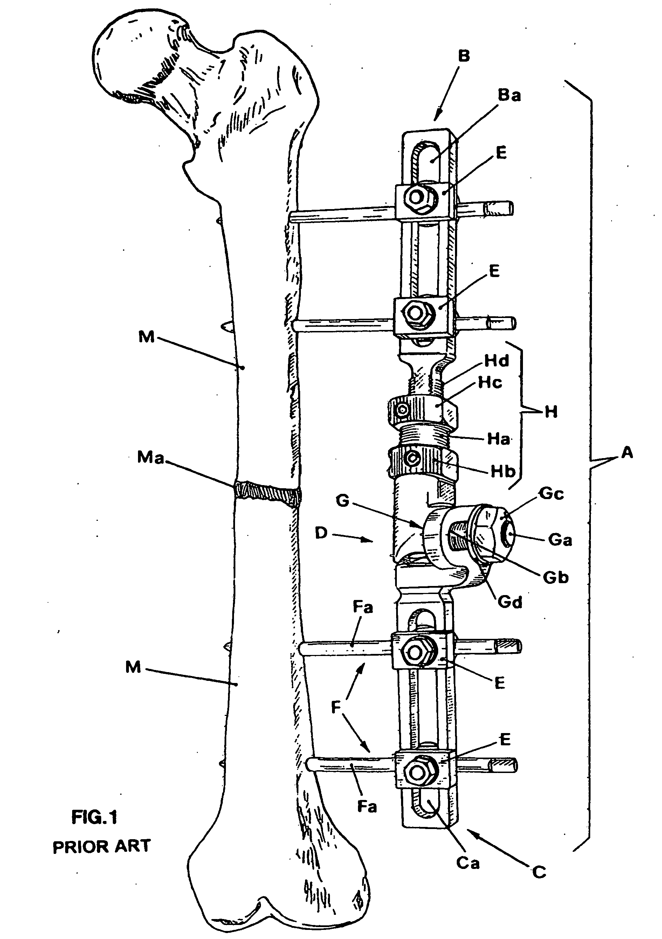

More particularly, an all-purpose external fixator of known type that is widely used to create the above-mentioned implants is illustrated in the axonometric view in fig. 1, where it is globally indicated by the letter **A;** it basically comprises a load-bearing rod **B** and a second load-bearing rod **C,** both developing mainly in the longitudinal plane, and connected together by means of a central member **D.**

As shown in the figure, each of the load-bearing rods **B, C** has a longitudinal slot **Ba, Ca** that slidingly contains one or more clamps **E** with associated connection elements **F** consisting of screws **Fa** that are inserted in the bone stumps **M.**

The load-bearing rods **B, C** are connected together by means of the above-mentioned central member **D,** which enables the former to be arranged in different positions because it comprises:
- an articulated joint **G** enabling the offsetting and reciprocal rotation of the load-bearing rods **B, C;**
- a screw unit **H** for displacing the load-bearing rods **B, C** closer to or further away from one another.

During the implantation of the fixator, the surgeon takes action on the screw unit **H** and the articulated joint **G** to adjust the distance, offset, angle and reciprocal rotation of the load-bearing rods **B, C.**

The screw unit **H** also enables the patient to progressively adjust the distance between the load-bearing rods after the application of the implant, and thus facilitate the optimal formation of the bone callus at the site of the fracture **Ma.**

The above-described all-purpose external fixator, the structural characteristics of which are also seen in other all-purpose external fixators available on the market, has a number of acknowledged drawbacks, however, as well as the previously-mentioned advantages.

In particular, a first drawback consists in that it is impossible to maintain the coaxial or offset alignment of the load-bearing rods **B, C** when action is taken to adjust the angle of inclination of the articulated joint **G,** or to maintain the relative angle of inclination between the load-bearing rods when action is taken on the articulated joint **G** to adjust its coaxial or offset alignment.

In fact, because the articulated joint comprises a screw **Ga** projecting from the central member **G** that is contained in a slot **Gb** in which the screw **Ga** is locked by means of a nut **Gc** and a washer **Gd,** whenever the nut **Gc** is loosened, the screw-slot pair becomes unstable and both the axial position, and the angle of inclination between the load-bearing rods **B, C** are consequently lost.

This makes it impossible to independently adjust the angle of inclination and the offset between the load-bearing rods.

Another drawback lies in that it is impossible to keep the angle through which the load-bearing rods are reciprocally rotated in relation to a plane crosswise to their longitudinal axis constant whenever action is taken on the screw unit **H** to modify the axial distance between said load-bearing rods.

In fact, the screw unit, which comprises a threaded sleeve **Ha** with a threaded ring nut **Hb** and a threaded counter ring nut **He** which contain a screw **Hd** belonging to one of the load-bearing rods (the load-bearing rod **C** in the case in point), is obviously unable to guarantee the stability of the angle through which the load-bearing rods are reciprocally rotated on a plane crosswise to their longitudinal axis when action is taken on the ring nuts to modify the axial distance between the load-bearing rods.

This makes it impossible to independently adjust the distance between the load-bearing rods and their reciprocal angle of rotation in relation to the plane crosswise to their longitudinal axis.

Another drawback lies in the fact that all the different load-bearing rod locking positions permitted by the articulated joint are defined by juxtaposed front knurling on the washer, slot and central member, which means that the locked positions of the load-bearing rods can be only varied in discrete quantities, not continuously, and this restricts the range of adjustments achievable.

Another drawback lies in that the means of connection between the component parts of the fixator do not have structural features suitable for enabling a controlled fine adjustment of their displacements and locking positions, and in that the fixator affords no useful references to help identify precise adjustment positions and thereby obtain calibrated locking conditions.

It is consequently impossible to adjust the means of connection between the parts comprising the fixator using screwing means with controlled-torque or positioning-control regulators in order to obtain precise adjustments.

The fact that it is impossible to adjust the device precisely carries the further drawback that all adjustments must be made by hand and in a sensory manner, so the validity of the fixator's assembly depends on the ability of the surgeon.

Finally, in the types of fixator that include a shock-absorbing device to enable a controlled and adjustable deflection of the load-bearing rods, another drawback lies in that modifying the load required to enable said deflection entails the need to substitute the deflection elements comprising the shock absorber device.

The fact that this substitution has to be done by specialized personnel interferes with the speedy performance of the procedure, which cannot always be done immediately due to difficulties in obtaining spare parts and/or in gaining access to a specialist.

Another known external fixator is disclosed in the document WO 88/05287 A, in which the two load-bearing rods are joined to one another by spherical connections, but the described joint has substantially the same drawbacks mentioned above.

The present invention aims to overcome all the above-listed drawbacks.

In particular, a first object of the invention is to realize an all-purpose external fixator for use in the field of orthopedic traumatology that enables the reciprocal angle of inclination of the load-bearing rods to be adjusted by modifying the inclination of each rod independently of the other.

Another object achieved by the fixator of the invention is that the inclination of each of the load-bearing rods can be adjusted independently of any other adjustments.

Another object achieved by the fixator of the invention is that the axial distance between the load-bearing rods is adjustable independently of any other adjustments.

Another object achieved by the fixator of the invention is the opportunity to adjust the angle of reciprocal rotation of the load-bearing rods in a plane crosswise to their longitudinal axis independently of any other adjustments.

Another object of the invention is for the fixator to enable a fine adjustment of the reciprocal angle of rotation of the load-bearing rods in a plane crosswise to their longitudinal axis.

Another object achieved by the fixator of the invention lies in that the angle of inclination between the load-bearing rods, their axial distance and their reciprocal angular rotation in a plane crosswise to their longitudinal axis can each be adjusted independently of the others.

Another object achieved lies in that the adjustments of the fixator can be calibrated by the operator using operating means complete with controlled-torque and/or positioning-control regulators.

Another, not necessarily last object achieved lies in that the fixator of the invention allows for the elastic force of the shock absorber device inserted between the load-bearing rods to be adjusted without having to replace any component parts of the shock absorber device.

The above-mentioned objects are achieved by an all-purpose external fixator for use in the field of orthopedic traumatology, the receiver features of which are in accordance with claim 1.

According to one embodiment, each of the articulated joints is of the hinged type and comprises a shaped head at each end of the central member, contained inside a fork provided at the end of each load-bearing rod.

Thus, these hinged joints enable the angle of inclination of the load-bearing rods to be modified independently of one another.

The central member comprises a sleeve and a stem coaxial with one another, whose angular position in relation to a plane crosswise to their longitudinal axis can be varied by toothed means that can be locked in different positions.

The load-bearing rods can thus be rotated reciprocally around their longitudinal axis and locked in different angular positions independently of one another.

According to another embodiment, the angular position between the sleeve and the stem can be varied continuously using toothed regulating means comprising a toothed crown engaging with a toothed pinion that can be operated using controlled-torque or positioning-control regulating means.

According to another embodiment, the fixator is also provided with shock absorbing means that enable a deflecting movement of the load-bearing rods.

In all the embodiments mentioned above, the means of connection between the component parts of the fixator are complete with operating devices that can be associated with motorized means, e.g. screwdrivers with controlled-torque or positioning-control regulators, to enable fine adjustments to be achieved.

As a consequence, the fixator advantageously enables all the adjustments to be made independently of one another.

Equally advantageously, each adjustment does not interfere with other, previously- or subsequently-performed adjustments.

Another advantage lies in the opportunity to obtain fine adjustments of the component parts of the fixator, thereby improving its performance.

The aforesaid objects and advantages emerge more clearly from the following description of preferred embodiments, provided here as a non-limiting example with reference to the attached drawings, wherein:
- fig. 1 shows an axonometric view of a fixator according to the known state of the art;
- fig. 2 shows a longitudinal view of the fixator of the invention;
- fig. 3 shows an enlarged axonometric longitudinal sectional view of a detail of the fixator of the invention shown in fig. 2;
- fig. 4 shows a longitudinal sectional view of the fixator of fig. 2 along the line IV-IV;
- fig. 5 shows a cross-sectional view of the fixator of fig. 2 along the line V-V;
- fig. 6 shows a cross-sectional view of the fixator of fig. 2 along the line VI-VI;
- figs. 7 to 13 show axonometric views of details of the fixator of the invention;
- fig. 14 shows a longitudinal view of another embodiment of the fixator of the invention;
- fig. 15 shows a longitudinal sectional view of the embodiment of the fixator of the invention shown in fig. 14 along the line XV-XV;
- fig. 16 shows a cross-sectional view of the embodiment of the fixator of the invention shown in fig. 14 along the line XVI-XVI;
- figs. 17 and 18 show axonometric views of details of the embodiment of the fixator of the invention shown in fig. 14;
- fig. 19 shows an enlarged detail of another embodiment of the fixator of the invention;
- fig. 20 shows an enlarged axonometric longitudinal sectional view of a detail of another embodiment of the fixator of the invention.

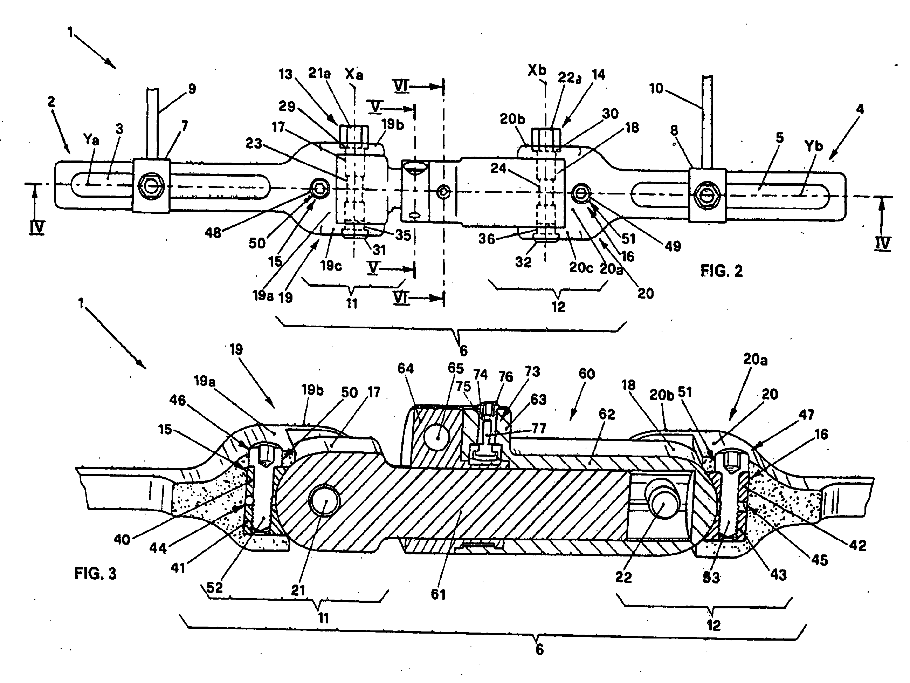

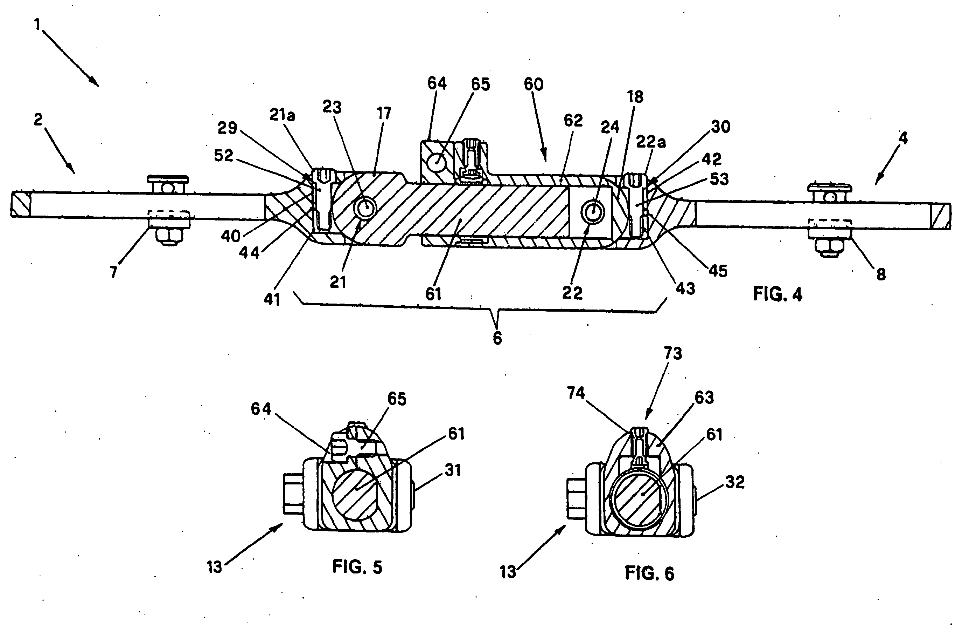

The external bone stump fixator forming the object of the invention is shown in a longitudinal view in fig. 2, in a longitudinal sectional view in fig. 4, and in an enlarged axonometric longitudinal sectional view in fig. 3, globally indicated by the numeral **1.**

It comprises a first load-bearing rod **2** identifying a first longitudinal axis **Ya** along which there is a first longitudinal slot **3,** and a second load-bearing rod **4** identifying a second longitudinal axis **Yb** along which there is a second longitudinal slot **5,** and a central member lying mainly in the longitudinal plane and globally indicated by the numeral **6,** that connects the two load-bearing rods **2, 4** together.

One or more clamps **7, 8** are slidingly coupled respectively in each of the slots, **3, 5** for holding screw connection elements **9, 10** for inserting in the bone stumps being connected together.

The clamps **7, 8** and the screw connection elements **9, 10** belong to the known state of the art and can be realized in various configurations and shapes suitable for enabling a better positioning of the former along the slots in the rods and a better grip on the bone stumps for the latter.

According to the invention, each end of the central member **6** co-operates with a corresponding end of the load-bearing rods to define articulated joints **11, 12,** each of which comprises means **13, 14** for adjusting and means **15, 16** for locking the reciprocal position of the load-bearing rods **2, 3** with respect to the central member **6.**

In particular, each of the above-mentioned articulated joints **11, 12** is of the hinged type and comprises a shaped head **17, 18** belonging to the central member **6** that is contained inside a fork **19, 20** belonging to each of the load-bearing rods **2, 4,** and consisting of a fork stem **19a, 20a** with a first prong **19b, 20b** and a second prong **19c, 20c** lying parallel to one another.

Clearly, in another embodiment, each shaped head may be obtained in the end of each of said load-bearing rods, while each of said forks may be obtained in the end of the central member.

Each shaped head **17, 18** and corresponding fork **19, 20** are revolvingly connected to one another by the above-mentioned adjustment means **13, 14** co-operating with the above-mentioned locking means **15, 16** to enable the various positions of the fixator to be established.

Each of the adjustment means **13, 14,** as shown in figs. 3, 7 and 8, comprises a revolving pin **21, 22** identifying a crosswise axis **Xa, Xb** at right angles to the axis **Ya, Yb** of the load-bearing rod **2, 3,** which engages in a crosswise through hole **23, 24** provided both in the shaped head **17, 18** and in the fork **19, 20.**

The pin **21, 22** has an operating head **21a, 22a** at one end and, at the other end, a coaxial threaded hole **25, 26** in the revolving pin **21, 22** for containing a fixing screw **27, 28.**

In particular, the operating head **21a, 22a** has a polygonal profile and is contained in a corresponding seat **29, 30** with a polygonal profile provided in the first prong 19b, 20b of the fork **19, 20,** as shown in fig. 2.

Thus, when the operating head **21a, 22a** is rotated, it induces a corresponding rotation of the respective load-bearing rod **2, 3,** thereby modifying the latter's angle of inclination with respect to the central member **6.**

The fixing screw **27, 28** has a head **31, 32,** which abuts against the second prong **19c, 20c** of the fork **19, 20** and which has a collar **33, 34** that engages in a hole **35, 36** provided in the second prong **19c, 20c.**

Thus, when the fixing screw **27, 28** is loosened, maneuvering the operating head **21a, 22a** makes the pin **21, 22** and the load-bearing rods **2, 3** rotate around the axis **Xa, Xb,** thereby independently modifying the angle of inclination of each of the load-bearing rods with respect to the central member and thus achieving one of the objects of the invention.

As shown particularly in figs. 3, 4 and 9, the locking means **15, 16** comprise a pair of shaped jaws **40, 41; 42, 43** lying opposite one another and contained in a seat **44, 45** in the stem **19a, 20a** of the fork **19, 20,** with shaped surfaces **40a, 41 a; 42a, 43a** in contact with the shaped head **17, 18.**

The shaped jaws **40, 41; 42, 43** are also associated with an operating screw **46, 47** that has an operating head **48, 49** inserted in a recess **50, 51** provided in the stem **19a, 20a** of the fork **19, 20** and a threaded body **52, 53** inserted in a through hole **40b, 42b** provided in the first shaped jaw **40, 42** and with its threaded end screwed into a threaded hole **41c, 43c** provided in the second jaw **41, 43.**

Thus, turning the screws **46, 47** forces the shaped surfaces **40a, 41a, 42a, 43a** of the jaws against the shaped heads and thereby locks the joint and consequently also each load-bearing rod in relation to the central body in the position established by means of the previously-described adjustment means.

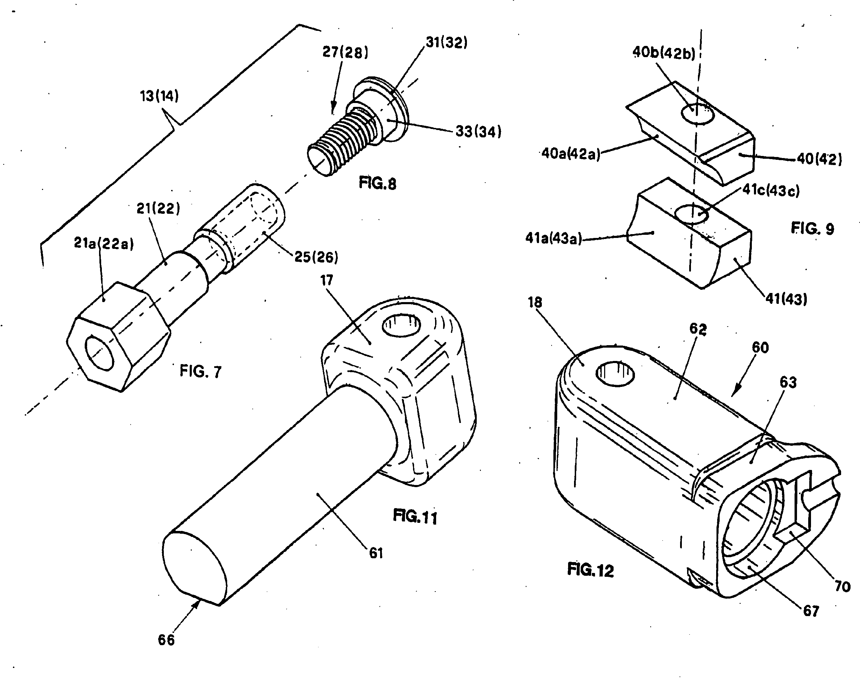

As shown particularly in figs. 3 and 4, the central member **6** comprises a sleeve **60** provided at one end with a first shaped head **18,** in which a stem **61** (also shown in fig. 11) slidingly engages, with a second shaped head at the other end **17.**

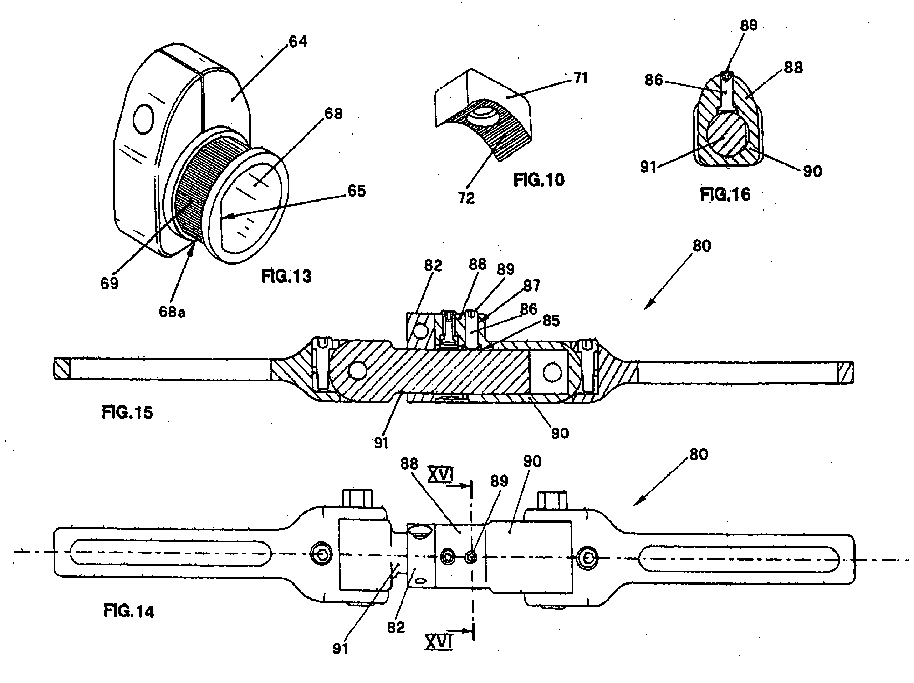

The sleeve **60** comprises a tubular member **62** (also shown in fig. 12) with the previously-mentioned first shaped head **18** at one end and, at the opposite end, a ring-shaped flange **63** (shown in detail in fig. 6) with a juxtaposed separate locking clamp **64** with a C-shaped crosswise profile, complete with a tangential locking screw **65,** as shown in fig. 5 and also in fig. 13.

On the inside of the locking clamp **64** there is also a longitudinal reference plane **65** abutting against a longitudinal reference plane **66** provided on the outside of the stem **61** to enable the sliding longitudinal displacement of the stem **61** inside the sleeve **60** and thus enable the elongation of the fixator.

Another object of the invention is thus achieved, in that the load-bearing rods can be separated from one another, independently of each other and of any other adjustments.

As shown particularly in fig. 12, the ring-shaped flange **63** has a ring-shaped seat **67** in which a ring-shaped appendage **68** on the locking clamp **64** (shown in fig. 13) engages, the outer lateral surface **68a** of said appendage bearing a set of teeth **69.**

The previously-mentioned ring-shaped flange **63** also has a recess **70** for containing a toothed sector **71** (shown in fig. 10), the teeth **72** of which engage with the set of teeth **69.**

The toothed sector 71 (shown particularly in figs. 3, 4, 6 and 10) is associated with screw fixing means **73** that are also coupled to the ring-shaped flange **63** and that comprise:
- a first screw **74** engaging in a threaded hole **75** in the ring-shaped flange **63** and complete with an operating head **76;**
- a coaxial second screw **77** coupled in the first screw **74** on the side opposite the operating head **76,** the end **78** of which is coupled to the toothed sector **71.**

Suitably operating the screw fixing means **73** therefore makes the teeth **72** of the toothed sector **71** engage with the teeth **69** provided on the ring-shaped appendage **68** and thus ensures that the sleeve **60** remains integrally attached to the stem **61.**

From the operational point of view, when the locking clamp **64** comprising the sleeve **60** is locked in place by closing the tangential tightening screw **65** and the toothed sector **71** engages with the set of teeth **69** on the ring-shaped appendage **68** of the locking clamp **64,** the sleeve **60** and the stem **61** are mutually locked in place, preventing any reciprocal rotation around their common longitudinal axis or any axial sliding between the two.

In fact, the engagement between the toothed sector **71** and the set of teeth **69** integrally attaches the ring-shaped flange **63** to the locking clamp **64,** and the interference between the reference plane **65** and the longitudinal reference plane **66** prevents any rotation of the stem **61** in the sleeve **60,** while the closure of the locking screw **65** prevents their reciprocal axial displacement in the longitudinal direction.

To reciprocally rotate the sleeve **60** and the stem **61,** and thereby modify the angular position of the load-bearing rods **2, 4,** the tangential locking screw **65** of the clamp **64** simply needs to be loosened so as to disengage the toothed sector **71** from the set of teeth **69** on the ring-shaped flange **63** can be removed.

Then the sleeve **60** and the stem **61** can be manually rotated with respect to one another.

After completing any such adjustment, the position reached is locked by tightening the tangential locking screw **65** of the locking clamp **64** and the toothed sector **71** is locked in place by tightening the screw fixing means **73.**

Thus, the object of enabling the reciprocal rotation of the load-bearing rods and the consequent modification of their angular position with respect to a plane at right angles to their longitudinal axis is also achieved.

Moreover, this adjustment can be made independently of any other adjustments and without affecting any such other adjustments.

An alternative embodiment of the fixator of the invention is shown in fig. 14, and in another view in fig. 15, where it is globally indicated by the numeral **80.**

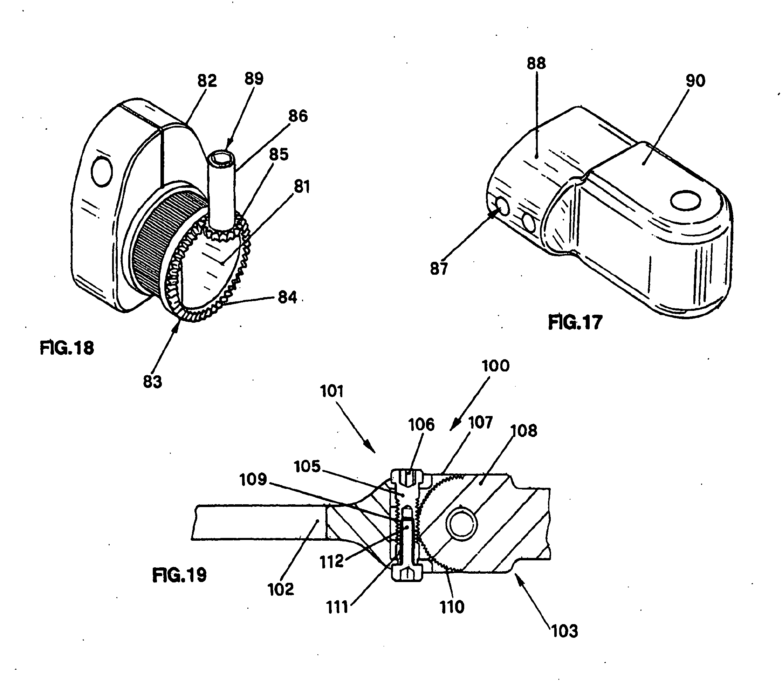

It differs from the previously-described embodiment in that the ring-shaped appendage **81** of the locking clamp **82** (shown in fig. 18) has a set of teeth **84** on the ring-shaped front surface **83** that engages with a pinion **85** associated with the end of an operating shaft **86** inserted in a hole **87** in the ring-shaped flange **88** (as shown in fig. 16).

At the opposite end to the pinion **85,** the operating shaft **86** has an operating head **89** for coupling with operating means for use by the operator to rotate the pinion **85** so as to obtain controlled fine rotational adjustments of the sleeve **90** with respect to the stem **91.**

Said adjustment is obtained in much the same way as explained previously, the only difference being that the reciprocal rotation of the sleeve **90** and stem **91** is obtained by rotating the operating shaft **86** of the pinion **85** instead of being done directly by hand.

The angular position of the sleeve **90** and stem **91** can thus be changed, for instance, using controlled-torque or positioning-control adjusting means connected to the operating head **89.**

A fine adjustment can consequently be obtained and this fulfills another of the objects of the invention.

Another embodiment of the fixator of the invention is shown in fig. 19, where it is globally indicated by the numeral **100.**

It only differs from the previously-described embodiments in that it comprises an adjustment device, globally indicated by the numeral **101,** for the precision reciprocal rotation of each load-bearing rod **102** in relation to the central member **103** in line with each articulated joint **104.**

In particular, each adjustment device **101** comprises a pin **105** with an operating head **106** positioned so as to pass through the fork **107** that, together with the shaped head **108,** constitutes the articulated joint **104.**

On the outside of the pin **105** there is a first set of teeth **109** that engages with a corresponding second set of teeth **110** on the outer surface of the shaped head **108.**

At the end opposite the operating head **106,** the pin 105 has an axial threaded hole **111** for containing a fixing screw **112** for preventing the pin from being separated from the articulated joint.

Manual or motorized operating means are applied to the operating head **106** to rotate the pin **105** to induce the rotation of the load-bearing rod **102** with respect to the central member **103.**

The operation and structural design of the adjustment device **101** are basically those of a worm screw and helical wheel type, which are characteristically irreversible.

The position reached by the load-bearing rod **102** after the adjustment has been made is consequently maintained so, in this embodiment of the invention, the locking means needed in the other embodiments described herein may be omitted.

Said alternative embodiment thus achieves the object of enabling a substantially micrometric, fine rotational adjustment of the load-bearing rods with a transmission ratio determined by the pitch of the teeth.

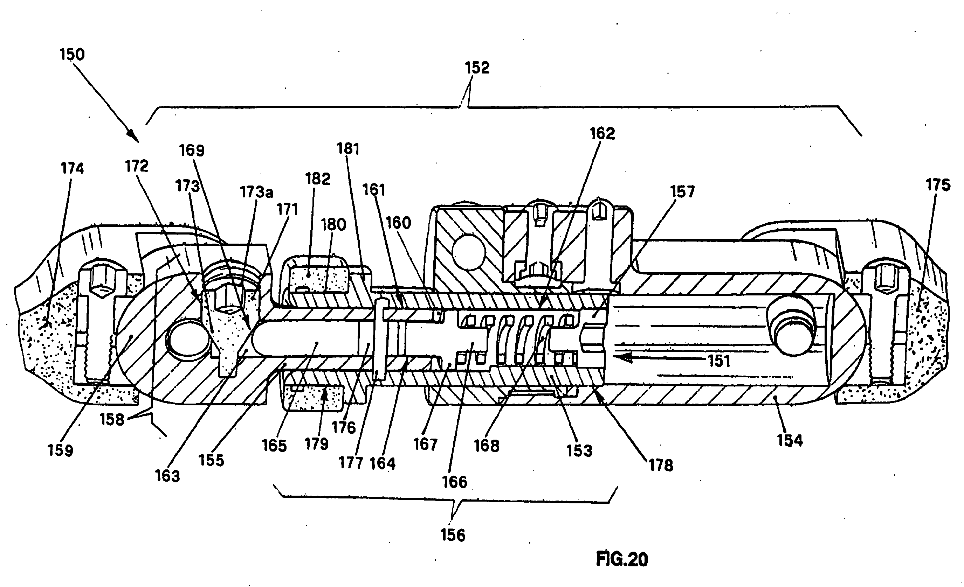

Another embodiment of the fixator of the invention is shown in fig. 20, where it is globally indicated by the numeral **150.**

It differs from the previously-described embodiment in that it comprises a shock absorber device **151** associated with the central member **152.**

In particular, the shock absorber device **151** can be applied to any of the previously-described embodiments of the fixator, not only to the embodiment described as an example herein.

The shock absorber device **151** comprises a tubular member **153** coaxially inserted between the sleeve **154** and the stem **155** forming the central member **152,** and containing in turn an elastic assembly **156** that interacts between the end **157** of the tubular member **153** and adjustment means **158** contained inside the shaped head **159** of the stem **155.**

The tubular element **153** contains a ring-shaped diaphragm **160** defining a first chamber **161** that slidingly contains the stem **155** and a coaxial second chamber **162** aligned in the direction of the first chamber **161** delimited by the end **157.**

The elastic assembly **156** is contained partly inside the second chamber **162** and partly inside a coaxial hole **163** in the stem **155.**

Moreover, the elastic assembly **156** comprises a sliding rod **164** of which a first stretch **165** lies coaxially inside the hole **163** in the stem **155** and a second stretch **166** lies coaxially inside the second chamber **162,** where said first stretch **165** and second stretch **166** are separated from one another by a counterflange **167** coaxial with the rod **164** and contained inside the second chamber **162.**

The elastic assembly **156** also comprises a helical spring **168** which is contained, as shown, inside the second chamber **162** and on the outside of the second stretch **166** of the sliding rod **164,** and which co-operates by contact with the counterflange **167** and the end **157.**

Moreover, the first stretch **165** of the sliding rod **164** has a rounded end **169** that cooperates in contact with the adjustment means **158.**

The adjustment means **158** comprise an adjusting screw **171** contained inside a threaded hole **172** in the shaped head **159,** with a conical body **173** placed in contact with the rounded end **169** of the sliding rod **164.**

An operating head **173a** on the adjusting screw **171** enables it to be screwed down or unscrewed in order to modify the position of the conical body **173.**

This induces an axial force on the sliding rod **164** and tightens or loosens the helical spring **168,** thereby modifying the value of the axial force that enables the reciprocal displacement of the load-bearing rods **174, 175** when they are loaded.

To govern said displacement, there is a through slot **176** in the first stretch **165** of the sliding rod **164** that contains a transversal reference pin **177** in the tubular member **153.**

Moreover, the tubular member **153** comprises a first part **178** partially engaging inside the sleeve **154** and a second part **179** with an outer thread **180** that are separated from one another by a ring-shaped flange **181.**

A threaded ring nut **182** is coupled to the outer thread **180** and serves as a limiting device to adjust the range of motion of the load-bearing rods **174, 175** permitted by the shock absorber device **151.**

Taking action on the adjustment means **158** thus enables a variation in the elastic force of reciprocal deflection between the load-bearing rods **174, 175,** as well as in their range of motion, by modifying the position of the threaded ring nut **182** to obtain an optimal loading of the fractured area between the bone stumps.

This enables the optimal formation of the bone callus and a consequent rapid recovery of the function of the fixated area, thereby achieving another object of the invention.

Based on the above considerations, the fixator of the invention clearly achieves all the previously-stated objects.

Particularly during the executive development phase, the fixator of the invention may undergo changes and further embodiments differing from those illustrated and described herein that shall also be covered by the present patent if they come within the scope of the following claims.

Where technical features mentioned in any claim are followed by reference signs, those reference sings have been included for the sole purpose of increasing the intelligibility of the claims and accordingly such reference signs do not have any limiting effect on the interpretation of each element identified by way of example by such reference signs.

## Claims

1. External bone stump fixator (1; 80; 100; 150) for use in orthopedics of the type comprising:
- a first load-bearing rod (2; 102; 174) identifying a first longitudinal axis (Ya) containing a first longitudinal slot (3);
- a second load-bearing rod (4; 175) identifying a second longitudinal axis (Yb) containing a second longitudinal slot (5);
- one or more clamps (7, 8) engaging slidingly in each of said slots (3, 5), for holding connection elements (9, 10) with screws for inserting in said bone stumps;
- a central member (6; 103; 152) developing mainly in the longitudinal direction and connected to both of said load-bearing rods (2, 4; 102; 174, 175),
each end of said central member (6; 103; 152) co-operating with a corresponding endof said load-bearing rods (2, 4; 102; 174, 175) to define articulated joints (11, 12; 104), each of which comprises means (13, 14) for adjusting and means (15, 16) for locking the reciprocal position of said load-bearing rods (2, 4; 102; 174, 175) and of said central member (6; 103; 152),
**characterized in that** each of said articulated joints (11, 12; 104) is of the hinged type and comprises a shaped head (17, 18; 108; 159) that fits into a fork (19, 20; 107) identified by a fork stem (19a, 20a) with a first prong (19b, 20b) and a second prong(19c, 20c) lying parallel to one another, said shaped head (17, 18; 108; 159) and said fork (19, 20; 107) being revolvingly connected to one another by said adjustment means (13, 14) and interacting reciprocally by means of the interposition of said locking means (15, 16).

2. External fixator (1; 80; 100; 150) according to claim 1, **characterized in that** each of said shaped heads (17, 18; 108; 159) comprising each of said articulated joints (11, 12; 104) is obtained in each end of said central member (6; 103; 152) and each of said forks (19, 20; 107) is obtained in the end of each of said load-bearing rods (2, 4; 102; 174, 175).

3. External fixator (1; 80; 100; 150) according to claim 1; **characterized in that** each of said adjustment means (13, 14) comprises a revolving pin (21, 22) that is coupled inside a transversal through hole (23, 24) in said shaped head (17, 18; 108; 159) and in said fork (19, 20; 107), and has an operating head (21a, 22a) at one end and, at the opposite end, a threaded hole (25, 26) coaxial to said revolving pin (21, 22), which contains a fixing screw (27, 28).

4. External fixator (1; 80; 100; 150) according to claim 3, **characterized in that** said operating head (21a, 22a) has a polygonal profile and is contained inside a corresponding seat (29, 30) with a polygonal profile in said first prong (19b, 20b) of said fork (19, 20; 107).

5. External fixator (1; 80; 100; 150) according to claim 3, **characterized in that** said fixing screw (27, 28) has a head (31, 32) abutting against said second prong (19c, 20c) and a collar (33, 34) that engages inside a hole (35, 36) in said second prong (19c, 20c).

6. External fixator (1; 80; 100; 150) according to claim 1, **characterized in that** said locking means (15, 16) comprise a pair of opposite shaped jaws (40, 41; 42, 43) contained in a recess (44, 45) in said stem (19a, 20a) of said fork (19, 20), which are placed in contact with said shaped head (17, 18; 108; 159) and are associated with an operating screw (46, 47) for forcing said pair of jaws (40, 41; 42, 43) against said shaped head (17,18; 108; 159).

7. External fixator (1; 80; 100; 150) according to claim 6, **characterized in that** said operating screw (46, 47) comprises an operating head (48, 49) inserted in a recess (50, 51) in said stem (19a, 20a) of said fork (19, 20; 107) and placed at one end of a threaded body (52, 53) inserted in a through hole (40b, 42b) in a first (40, 42) of said shaped jaws and having its threaded end screwed into a threaded hole (41c, 43c) in a second (41, 43) of said jaws.

8. External fixator (1; 80; 100; 150) according to claim 2, **characterized in that** said central member (6; 103; 152) comprises:
- a sleeve (60) with a first shaped head (18) at one end;
- a stem (61; 155) slidingly engaging in said sleeve (60) with a second shaped head (17; 159) at one end and with a longitudinal reference plane (66) on the outside.

9. External fixator (1; 80; 100; 150) according to claim 8, **characterized in that** said sleeve (60) comprises:
- a tubular member (62) with said first shaped head (18) at one end and a ring-shaped flange (63) at the opposite end;
- a locking clamp (64; 82) with a C-shaped crosswise profile abutting against said ring-shaped flange (63; 88), with a tangential locking screw (65).

10. External fixator (1; 80; 100; 150) according to claim 9, **characterized in that** said locking clamp (64; 82) has a longitudinal reference plane (65) on the inside facing and in sliding contact with said longitudinal reference plane (68) on the outside of said stem (61; 105).

11. External fixator (1; 80; 100; 150) according to claim 9, **characterized in that** said ring-shaped flange (63; 88) has a ring-shaped recess (67) in which a ring-shaped appendage (68; 81) on said locking clamp (64; 82) engages, with a set of teeth (69) on the outer lateral surface (68a) that engage in corresponding teeth (72) in a toothed sector (71) contained in a recess (70) in said ring-shaped flange (63).

12. External fixator (1; 80; 100; 150) according to claim 11, **characterized in that** said toothed sector (71) is associated with screw fixing means (73) coupled inside said ring-shaped flange (63).

13. External fixator (1; 80; 100; 150) according to claim 12, **characterized in that** said screw fixing means (73) comprise:
- a first screw (74) coupled inside a threaded hole (75) in said ring-shaped flange (63) and complete with an operating head (76);
- a coaxial second screw (77) coupled inside said first screw (74) on the side opposite said operating head (76) with its end (78) coupled to said toothed sector (71).

14. External fixator (80) according to claim 11 **characterized in that** said ring-shaped appendage (81) on said locking clamp (82) has a set of teeth (84) on its ring-shaped front surface (83) that engage with a pinion (85) associated with the end of an operating shaft (86) inserted in a hole (87) in said ring-shaped flange (88).

15. External fixator (80) according to claim 14, **characterized in that** said operating shaft (86) has an operating head (89) at the end opposite said pinion (85) for coupling with operating means.

16. External fixator (100) according to claim 1, **characterized in that** it comprises a device (101) for adjusting the reciprocal rotation of each of said load-bearing rods (102) in relation to said central member (103), said adjustment device (101) being positioned in line with each of said articulated joints (104).

17. External fixator (100) according to claim 16, **characterized in that** said adjustment device (101) comprises:
- a pin (105) fitted with an operating head (106) passing through said fork (107) and said shaped head (108) defining said articulated joint (104);
- a first set of teeth (109) provided peripherally around said pin (105);
- a second set of teeth (110) provided on the outer surface of said shaped head (108).

18. External fixator (100) according to claim 17, **characterized in that** said pin (105) opposite said operating head (106) has a threaded axial hole (111) containing a fixing screw (112).

19. External fixator (150) according to claim 8, **characterized in that** it comprises a shock-absorber device (151) associated with said central member (152).

20. External fixator (150) according to claim 19, **characterized in that** said shock-absorber device (151) comprises a tubular member (153) interposed coaxially between said sleeve (154) and said stem (155) and containing an elastic assembly (156) interacting between the end (157) of said tubular member (153) and adjustment means (158) contained inside a shaped head (159) of said central member (152).

21. External fixator (150) according to claim 20, **characterized in that** said tubular member (153) contains a ring-shaped diaphragm (160) that defines:
- a first chamber (161) slidingly containing said stem (155);
- a coaxial second chamber (112) delineated by said end (157) and aligned with said first chamber (161), said elastic assembly (106) being contained partially inside said second chamber (112) and partially in an axial hole (113) provided in said stem (105).

22. External fixator (150) according to claim 21, **characterized in that** said elastic assembly (156) comprises a sliding rod (164) of which a first stretch (165) lies coaxially inside said axial hole (163) in said stem (155) and a second stretch (166) lies coaxially inside said second chamber (162), said first stretch (165) and second stretch (166) being separated from one another by a counterflange (167) coaxial to said sliding rod (164) and contained inside said second chamber (162).

23. External fixator (150) according to claim 22, **characterized in that** said elastic assembly (156) also comprises a helical spring (168) contained inside said second chamber (162), around said second stretch (166) of said sliding rod (164) and cooperating by contact with said counterflange (167) and said end (157).

24. External fixator (150) according to claim 22, **characterized in that** said first stretch (165) of said sliding rod (164) has a rounded end (169) that cooperates by contact with said adjustment means 158).

25. External fixator (150) according to claim 24,**characterized in that** said adjustment means (158) comprise a regulating screw (171) contained inside a threaded hole (172) in said shaped head (159), with an operating head (173a) and a conical body (173) placed in contact with said rounded end (119) of said sliding rod (164).

26. External fixator (150) according to claim 22, **characterized in that** said first stretch (165) of said sliding rod (164) has a through slot (176) containing a reference pin (177) lying crosswise to said tubular member (153).

27. External fixator (150) according to claim 20, **characterized in that** said tubular member (153) has a first part (178) partially engaging inside said sleeve (154) and a second part (179) with an outer thread (180), the two parts being separated from one another by a ring-shaped flange (181).

28. External fixator (150) according to claim 27, **characterized in that** a threaded ring nut (182) is coupled to said outer thread (180) on said second part (179) of said tubular member (153).

## Patentansprüche

1. Fixateur externe (1; 80; 100; 150) für Knochenstümpfe zur orthopädischen Verwendung vom Typ, der Folgendes umfasst:
- einen ersten lasttragenden Stab (2; 102; 174), der eine erste Längsachse (Ya) definiert und einen ersten Schlitz (3) enthält;
- einen zweiten lasttragenden Stab (4; 175), der eine zweite Längsachse (Yb) definiert und einen zweiten Schlitz (5) enthält;
- eine oder mehrere Klemmen (7, 8) , die gleitend in jedem der Schlitze (3, 5) eingreifen, um Verbindungselemente (9, 10) mit Schrauben zur Einführung in die Knochenstümpfe zu halten;
- ein Mittelelement (6; 103; 152), das sich vorwiegend in Längsrichtung ausdehnt und mit beiden lasttragenden Stäben (2, 4; 102; 174, 175) verbunden ist,
wobei jedes Ende des Mittelelements (6; 103; 152) mit einem entsprechenden Ende der lasttragenden Stäbe (2, 4; 102; 174, 175) zusammenwirkt, um Gelenksverbindungen (11, 12; 104) zu definieren, von denen jede Mittel (13, 14) zum Einstellen und Mittel (15, 16) zum Sperren der gegenseitigen Stellung der lasttragenden Stäbe (2, 4; 102; 174, 175) und des Mittelelements (6; 103; 152) aufweisen,
**dadurch gekennzeichnet, dass** jede der Gelenksverbindungen (11, 12; 104) vom Scharniertyp ist und einen Formkopf (17, 18; 108, 159) umfasst, der in eine Gabel (19, 20; 107) passt, die von einem Gabelstiel (19a, 20a) mit einer ersten Zinke (19b, 20b) und mit einer zweiten Zinke (19c, 20c), die parallel zueinander liegen, definiert ist, wobei der Formkopf (17, 18; 108, 159) und die Gabel (19, 20; 107) durch das Einstellmittel (13, 14) drehbar miteinander verbunden sind und durch das Dazwischenanordnen des Sperrmittels (15, 16) gegenseitig wechselwirken.

2. Fixateur externe (1; 80; 100; 150) nach Anspruch 1), **dadurch gekennzeichnet, dass** jeder der Formköpfe (17, 18; 108, 159), den jede der Gelenksverbindungen (11, 12; 104) umfasst, in jedem Ende des Mittelelements (6; 103; 152) ausgebildet ist, und jede der Gabeln (19, 20; 107) im Ende eines jeden lasttragenden Stabs (2, 4; 102; 174, 175) ausgebildet ist.

3. Fixateur externe (1; 80; 100; 150) nach Anspruch 1), **dadurch gekennzeichnet, dass** jedes der Einstellmittel (13, 14) einen Drehzapfen (21, 22) umfasst, der im Inneren eines Durchgangslochs (23, 24), das transversal im Formkopf (17, 18; 108, 159) und in der Gabel (19, 20; 107) ausgebildet ist, gekoppelt ist und an einem Ende einen Betätigungskopf (21 a, 22a) und am gegenüberliegenden Ende ein Gewindeloch (25, 26) aufweist, das koaxial zum dem Drehzapfen (21, 22) ist und eine Fixierschraube (27, 28) enthält.

4. Fixateur externe (1; 80; 100; 150) nach Anspruch 3), **dadurch gekennzeichnet, dass** der Betätigungskopf (21 a, 22a) ein polygonales Profil aufweist und im Inneren eines entsprechenden Sitzes (29, 30) mit polygonalem Profil in der ersten Zacke (19b, 20b) der Gabel (19, 20; 107) aufgenommen ist.

5. Fixateur externe (1; 80; 100; 150) nach Anspruch 3), **dadurch gekennzeichnet, dass** die Fixierschraube (27, 28) einen Kopf (31, 32), der an der zweiten Zacke (19c, 20c) anstößt, sowie einen Bund (33, 34), der im Inneren eines Lochs (35, 36) in der zweiten Zacke (19c, 20c) eingreift, aufweist.

6. Fixateur externe (1; 80; 100; 150) nach Anspruch 1), **dadurch gekennzeichnet, dass** die Sperrmittel (15, 16) ein Paar aus gegenüberliegenden Formbacken (40, 41; 42, 43) umfassen, die in einer Ausnehmung (44, 45) im Stiel (19a, 20a) der Gabel (19, 20) aufgenommen sind, in Kontakt zum Formkopf (17, 18; 108, 159) angeordnet sind und mit einer Betätigungsschraube (46, 47) in Verbindung stehen, um das Paar aus Formbacken (40, 41; 42, 43) gegen den Formkopf (17, 18; 108, 159) zu drücken.

7. Fixateur externe (1; 80; 100; 150) nach Anspruch 6), **dadurch gekennzeichnet, dass** die Betätigungsschraube (46, 47) einen Betätigungskopf (48, 49) umfasst, der in einer Ausnehmung (50, 51) im Stiel (19a, 20a) der Gabel (19, 20; 107) eingeführt ist und an einem Ende eines Gewindekörpers (52, 53) angeordnet ist, der in einem Durchgangsloch (40b, 42b) in einer ersten (40, 42) der Formbacken eingeführt ist und dessen Gewindeende in ein Gewindeloch (41 c, 43c) in einer zweiten (41, 43) der Formbacken eingeschraubt ist.

8. Fixateur externe (1; 80; 100; 150) nach Anspruch 2), **dadurch gekennzeichnet, dass** das Mittelelement (6; 103; 152) Folgendes umfasst:
- eine Hülse (60) mit einem ersten Formkopf (18) an einem Ende;
- einen Stiel (61; 155), der gleitend in die Hülse (60) eingreift, mit einem zweiten Formkopf (17; 159) an einem Ende und einer Längsbezugsebene (66) an der Außenseite.

9. Fixateur externe (1; 80; 100; 150) nach Anspruch 8), **dadurch gekennzeichnet, dass** die Hülse (60) Folgendes umfasst:
- ein röhrenförmiges Element (62) mit dem ersten Formkopf (18) an einem Ende und einem ringförmigen Flansch (63) am gegenüberliegenden Ende;
- eine Sperrklemme (64; 82) mit einem C-förmigen Querschnittsprofil, die am ringförmigen Flansch (63; 88) anstößt und eine tangentiale Befestigungsschraube (65) aufweist.

10. Fixateur externe (1; 80; 100; 150) nach Anspruch 9), **dadurch gekennzeichnet, dass** die Sperrklemme (64; 82) eine Längsbezugsebene (65) an der Innenseite aufweist, die zur Längsbezugsebene (68) an der Außenseite des Stiels (61; 105) hinweist und in Gleitkontakt zu dieser ist.

11. Fixateur externe (1; 80; 100; 150) nach Anspruch 9), **dadurch gekennzeichnet, dass** der ringförmige Flansch (63; 88) eine ringförmige Ausnehmung (67) aufweist, in der ein ringförmiger Ansatz (68; 81) eingreift, der an der Sperrklemme (64; 82) ausgebildet ist und der an der äußeren Seitenoberfläche (68a) einen Satz Zähne (69) aufweist, die in entsprechende Zähne (72) eines gezahnten Bereichs (71), der in einer Ausnehmung (70) im ringförmigen Flansch (63) aufgenommen ist, eingreifen.

12. Fixateur externe (1; 80; 100; 150) nach Anspruch 11), **dadurch gekennzeichnet, dass** der gezahnte Bereich (71) mit Schraubfixiermitteln (73) im Inneren des ringförmigen Flanschs (63) gekoppelt ist.

13. Fixateur externe (1; 80; 100; 150) nach Anspruch 12), **dadurch gekennzeichnet, dass** die Schraubfixiermittel (73) Folgendes umfassen:
- eine erste Schraube (74), die im Inneren eines Gewindelochs (75) im ringförmigen Flansch (63) gekoppelt ist und mit einem Betätigungskopf (76) ausgestattet ist;
- eine koaxiale zweite Schraube (77), die im Inneren der ersten Schraube (74) an jener Seite, die dem Betätigungskopf (76) gegenüberliegt, gekoppelt ist, wobei ihr Ende (78) mit dem gezahnten Bereich (71) gekoppelt ist.

14. Fixateur externe (80) nach Anspruch 11), **dadurch gekennzeichnet, dass** der ringförmige Ansatz (81) der Sperrklemme (82) an seiner ringförmigen Vorderfläche (83) einen Satz Zähne (84) aufweist, die in ein Zahnrad (85) eingreifen, das mit dem Ende einer Betätigungswelle (86) in Verbindung steht, welche in einem Loch (87) im ringförmigen Flansch (88) eingeführt ist.

15. Fixateur externe (80) nach Anspruch 14), **dadurch gekennzeichnet, dass** die Betätigungswelle (86) an jenem Ende, das dem Zahnrad (85) gegenüberliegt, einen Betätigungskopf (89) zur Kopplung mit Betätigungsmitteln aufweist.

16. Fixateur externe (100) nach Anspruch 1), **dadurch gekennzeichnet, dass** er eine Vorrichtung (101) zum Einstellen der gegenseitigen Drehung eines jeden der lasttragenden Stäbe (102) in Bezug auf das Mittelelement (103) umfasst, wobei die Einstellvorrichtung (101) an jeder der Gelenksverbindungen (104) angeordnet ist.

17. Fixateur externe (100) nach Anspruch 16), **dadurch gekennzeichnet, dass** die Einstellvorrichtung (101) Folgendes umfasst:
- einen Zapfen (105), der mit einem Betätigungskopf (106) ausgestattet ist, der durch die Gabel (107) und den Formkopf (108), die die Gelenksverbindung (104) definieren, tritt;
- einen ersten Satz Zähne (109), der um den Umfang herum am Zapfen (105) bereitgestellt ist;
- einen zweiten Satz Zähne (110), der an der Außenoberfläche des Formkopfs (108) bereitgestellt ist.

18. Fixateur externe (100) nach Anspruch 17), **dadurch gekennzeichnet, dass** der Zapfen (105) gegenüber dem Betätigungskopf (106) ein axiales Gewindeloch (111) aufweist, das eine Fixierschraube (112) enthält.

19. Fixateur externe (150) nach Anspruch 8), **dadurch gekennzeichnet, dass** er eine Stoßdämpfungseinrichtung (151) aufweist, die mit dem Mittelelement (152) in Verbindung steht.

20. Fixateur externe (150) nach Anspruch 19), **dadurch gekennzeichnet, dass** die Stoßdämpfungseinrichtung (151) ein röhrenförmiges Element (153) umfasst, das koaxial zwischen der Hülse (154) und dem Stiel (155) angeordnet ist und eine elastische Anordnung (156) enthält, die zwischen dem Ende (157) des röhrenförmiges Elements (153) und Einstellmitteln (158), die im Inneren eines Formkopfs (159) des Mittelelements (152) aufgenommen sind, wechselwirkt.

21. Fixateur externe (150) nach Anspruch 20), **dadurch gekennzeichnet, dass** das röhrenförmige Element (153) eine ringförmige Membran (160) enthält, die Folgendes definiert:
- eine erste Kammer (161), in der der Stiel (155) gleitend aufgenommen ist;
- eine koaxiale zweite Kammer (112), die vom Ende (157) abgegrenzt ist und mit der ersten Kammer (161) ausgerichtet ist;
wobei die elastische Anordnung (106) teilweise im Inneren der zweiten Kammer (112) und teilweise in einem axialen Loch (113), das im Stiel (105) ausgebildet ist, aufgenommen ist.

22. Fixateur externe (150) nach Anspruch 21), **dadurch gekennzeichnet, dass** die elastische Anordnung (156) einen gleitenden Stab (164) umfasst, von dem ein erster Abschnitt (165) koaxial im Inneren des axialen Lochs (163) im Stiel (155) liegt und ein zweiter Abschnitt (166) koaxial im Inneren der zweiten Kammer (162) liegt, wobei der erste Abschnitt (165) und der zweite Abschnitt (166) durch einen Gegenflansch (167) voneinander getrennt sind, der koaxial zum gleitenden Stab (164) ist und im Inneren der zweiten Kammer (162) aufgenommen ist.

23. Fixateur externe (150) nach Anspruch 22), **dadurch gekennzeichnet, dass** die elastische Anordnung (156) ferner eine Spiralfeder (168) umfasst, die im Inneren der zweiten Kammer (162) aufgenommen ist, um den zweiten Abschnitt (166) des gleitenden Stabs (164) angeordnet ist und durch Kontakt mit dem Gegenflansch (167) und dem Ende (157) zusammenwirkt.

24. Fixateur externe (150) nach Anspruch 22), **dadurch gekennzeichnet, dass** der erste Abschnitt (165) des gleitenden Stabs (164) ein abgerundetes Ende (169) aufweist, das durch Kontakt mit den Einstellmitteln (158) zusammenwirkt.

25. Fixateur externe (150) nach Anspruch 24), **dadurch gekennzeichnet, dass** die Einstellmittel (158) eine Regulierschraube (171) umfassen, die im Inneren eines Gewindelochs (172) im Formkopf (159) aufgenommen ist und einen Betätigungskopf (173a) und einen konischen Körper (173) aufweist, der in Kontakt zum abgerundeten Ende (119) des gleitenden Stabs (164) angeordnet ist.

26. Fixateur externe (150) nach Anspruch 22), **dadurch gekennzeichnet, dass** der erste Abschnitt (165) des gleitenden Stabs (164) einen Durchgangsschlitz (176) aufweist, der einen Bezugszapfen (177) enthält, der quer zum röhrenförmigen Element (153) liegt.

27. Fixateur externe (150) nach Anspruch 20), **dadurch gekennzeichnet, dass** das röhrenförmige Element (153) einen ersten Teil (178), der teilweise im Inneren der Hülse (154) eingreift, und einen zweiten Teil (179) mit einem Außengewinde (180) aufweist, wobei die beiden Teile durch einen ringförmigen Flansch (181) voneinander getrennt sind.

28. Fixateur externe (150) nach Anspruch 27), **dadurch gekennzeichnet, dass** eine ringförmige Gewindemutter (182) mit dem Außengewinde (180) am zweiten Teil (179) des röhrenförmigen Elements (153) gekoppelt ist.

## Revendications

1. Fixateur externe (1; 80; 100; 150) de moignons osseux pour l'orthopédie du type comprenant:
- une première tige porteuse (2; 102; 174) identifiant un premier axe longitudinal (Ya) contenant une première fente longitudinale (3);
- une deuxième tige porteuse (4; 175) identifiant un deuxième axe longitudinal (Yb) contenant une deuxième fente longitudinale (5);
- un ou deux étaux (7, 8) s'accouplant de manière coulissante avec chacune desdites fentes (3, 5), pour fixer des éléments de connexion (9, 10) avec des vis pour l'insertion dans lesdits moignons osseux;
- un élément central (6; 103; 152) se développant principalement en direction longitudinale et relié avec les deux tiges porteuses (2; 4; 102; 174; 175),
chaque extrémité dudit élément central (6; 103; 152) coopérant avec une extrémité correspondante desdites tiges porteuses (2, 4; 102; 174, 175) pour définir des articulations (11, 12; 104), chacune de celles-ci comprend des moyens (13, 14) pour le réglage et des moyens (15, 16) pour bloquer la position réciproque desdites tiges porteuses (2, 4; 102; 174, 175) et dudit élément central (6; 103; 152),
**caractérisé en ce que** chaque articulation (11, 12; 104) est du type à charnière et comprend une tête galbée (17, 18; 108; 159) qui est accueillie dans une fourche (19, 20; 107) identifiée par une tige de fourche (19a, 20a) munie d'une première patte (19b, 20b) et d'une deuxième patte (19c, 20c) se disposant parallèle à l'autre, ladite tête galbée (17, 18; 108; 159) et ladite fourche (19, 20; 107) étant reliées entre elles de manière pivotante par lesdits moyens de réglage (13, 14) et intérageant réciproquement par l'interposition desdits moyens de blocage (15, 16).

2. Fixateur externe (1; 80; 100; 150) selon la revendication 1), **caractérisé en ce que** chacune desdites têtes galbées (17, 18; 108; 159) comprenant chacun desdites articulations (11, 12; 104) est réalisée sur chaque extrémité dudit élément central (6; 103; 152) et chacune desdites fourches (19, 20; 107) est réalisée sur l'extrémité de chacune desdites tiges porteuses (2, 4; 102; 174, 175).

3. Fixateur externe (1; 80; 100; 150) selon la revendication 1), **caractérisé en ce que** chacun des moyens de réglage (13, 14) comprend un pivot de rotation (21, 22) qui s'accouple dans un trou passant transversal (23, 24) réalisé dans ladite tête galbée (17, 18; 108; 159) et dans ladite fourche (19, 20; 107), et présente une tête de manoeuvre (21 a, 22a) sur une extrémité et, sur l'extrémité opposée, présente un trou fileté (25, 26) coaxial audit pivot de rotation (21, 22), accueillant une vis de fixation (27, 28).

4. Fixateur externe (1; 80; 100; 150) selon la revendication 3), **caractérisé en ce que** ladite tête de manoeuvre (21 a, 22a) présente un profil polygonal et est logée à l'intérieur d'un siège correspondant (29, 30) ayant un profil polygonal dans ladite première patte (19b, 20b) de ladite fourche (19, 20; 107).

5. Fixateur externe (1; 80; 100; 150) selon la revendication 3), **caractérisé en ce que** ladite vis de fixation (27, 28) présente une tête (31, 32) s'appuyant contre ladite deuxième patte (19c, 20c) et un collet (33, 34) qui s'accouple dans un trou (35, 36) réalisé dans ladite deuxième patte (19c, 20c).

6. Fixateur externe (1; 80; 100; 150) selon la revendication 1), **caractérisé en ce que** lesdits moyens de blocage (15, 16) comprennent une paire de mâchoires galbées opposées (40, 41; 42, 43) logées dans une cavité (44, 45) réalisée dans ladite tige (19a, 20a) de ladite fourche (19, 20), qui sont en contact avec ladite tête galbée (17, 18; 108; 159) et sont associées avec une vis de manoeuvre (46, 47) apte à forcer ladite paire de mâchoires (40, 41; 42, 43) contre ladite tête galbée (17, 18; 108; 159).

7. Fixateur externe (1; 80; 100; 150) selon la revendication 6), **caractérisé en ce que** ladite vis de manoeuvre (46, 47) comprend une tête de manoeuvre (48, 49) insérée dans une cavité (50, 51) réalisée dans ladite tige (19a, 20a) de ladite fourche (19, 20; 107) et positionnée sur une extrémité d'un corps fileté (52, 53) inséré dans un trou passant (40b, 42b) dans une première (40, 42) desdites mâchoires galbées et ayant son extrémité filetée vissée dans un trou fileté (41 c, 43c) realisé dans une deuxième (41, 43) desdites mâchoires.

8. Fixateur externe (1; 80; 100; 150) selon la revendication 2), **caractérisé en ce que** ledit élément central (6; 103; 152) comprend:
- un manchon (60) ayant une première tête galbée (18) sur une extrémité;
- une tige (61; 155) s'accouplant de manière coulissante dans ledit manchon (60) ayant une deuxième tête galbée (17; 159) sur une extrémité et un plan longitudinal de référence (66) à l'extérieur.

9. Fixateur externe (1; 80; 100; 150) selon la revendication 8), **caractérisé en ce que** ledit manchon (60) comprend:
- un élément tubulaire (62) ayant une première tête galbée (18) sur une extrémité et une bride annulaire (63) sur l'extrémité opposée;
- un étau de blocage (64; 82) ayant un profil transversal en forme de C s'appuyant contre ladite bride annulaire (63; 88) et ayant une vis tangentielle de blocage (65).

10. Fixateur externe (1; 80; 100; 150) selon la revendication 9), **caractérisé en ce que** ledit étau de blocage (64; 82) présente à l'intérieur un plan longitudinal de référence (65) en face et en contact à glissement avec ledit plan longitudinal de référence (68) réalisé sur la partie externe de ladite tige (61; 105).

11. Fixateur externe (1; 80; 100; 150) selon la revendication 9), **caractérisé en ce que** ladite bride annulaire (63; 88) présente une cavité annulaire (67) dans laquelle s'accouple une appendice annulaire (68; 81) réalisée sur ledit étau de blocage (64; 82) et ayant sur la surface latérale externe (68a) un jeu de dents (69) qui s'engage avec des dents correspondantes (72) dans un secteur denté (71) accueilli dans une cavité (70) réalisée dans ladite bride annulaire (63).

12. Fixateur externe (1; 80; 100; 150) selon la revendication 11), **caractérisé en ce que** ledit secteur denté (71) est associé avec des moyens de fixation à vis (73) accouplés à l'intérieur de ladite bride annulaire (63).

13. Fixateur externe (1; 80; 100; 150) selon la revendication 12), **caractérisé en ce que** lesdits moyens de fixation à vis (73) comprennent:
- une première vis (74) accouplée dans un trou fileté (75) réalisé dans ladite bride annulaire (63) et munie d'une tête de manoeuvre (76);
- une deuxième vis coaxiale (77) accouplée dans ladite première vis (74) sur le côté opposé à ladite tête de manoeuvre (76) et ayant son extrémité (78) accouplée audit secteur denté (71).

14. Fixateur externe (80) selon la revendication 11), **caractérisé en ce que** ladite appendice annulaire (81) sur ledit étau de blocage (82) présente un jeu de dents (84) sur sa surface annulaire avant (83) qui s'accouple avec un pignon (85) associé avec l'extrémité d'un arbre de manoeuvre (86) inséré dans un trou (87) réalisé dans ladite bride annulaire (88).

15. Fixateur externe (80) selon la revendication 14), **caractérisé en ce que** ledit arbre de manoeuvre (86) présente une tête de manoeuvre (89) sur l'extrémité opposée audit pignon (85) apte à s'accoupler avec des moyens de manoeuvre.

16. Fixateur externe (100) selon la revendication 1), **caractérisé en ce qu'**il comprend un dispositif (101) pour le réglage de la rotation réciproque de chacune desdites tiges porteuses (102) par rapport audit élément central (103), ledit dispositif de réglage (101) étant positionné en ligne avec chacune desdites articulations (104).

17. Fixateur externe (100) selon la revendication 16), **caractérisé en ce que** ledit dispositif de réglage (101) comprend:
- un pivot (105) muni d'une tête de manoeuvre (106) passant à travers ladite fourche (107) et ladite tête galbée (108) définissant ladite articulation (104);
- un premier jeu de dents (109) réalisé périphériquement autour dudit pivot (105);
- un deuxième jeu de dents (110) réalisé sur la surface externe de ladite tête galbée (108).

18. Fixateur externe (100) selon la revendication 17), **caractérisé en ce que** ledit pivot (105) opposé à ladite tête de manoeuvre (106) présente un trou axial fileté (111) contenant une vis de fixation (112).

19. Fixateur externe (150) selon la revendication 8), **caractérisé en ce qu'**il comprend un dispositif amortisseur (151) associé avec ledit élément central (152).

20. Fixateur externe (150) selon la revendication 19), **caractérisé en ce que** ledit dispositif amortisseur (151) comprend un élément tubulaire (153) interposé coaxialement entre ledit manchon (154) et ladite tige (155) et contenant un groupe élastique (156) intérageant entre l'extrémité (157) dudit élément tubulaire (153) et des moyens de réglage (158) logés à l'intérieur d'une tête galbée (159) dudit élément central (152).

21. Fixateur externe (150) selon la revendication 20), **caractérisé en ce que** ledit élément tubulaire (153) contient une membrane annulaire (160) qui définit:
- une première chambre (161) qui accueillit de manière coulissante ladite tige (155);
- une deuxième chambre coaxiale (112) délinéée par ladite extrémité (157) et alignée avec ladite première chambre (161),
ledit groupe élastique (106) étant accueilli partiellement dans ladite deuxième chambre (112) et partiellement dans un trou axial (113) réalisé sur ladite tige (105).

22. Fixateur externe (150) selon la revendication 21), **caractérisé en ce que** ledit groupe élastique (156) comprend une tige coulissante (164) de laquelle une première section (165) est positionnée coaxialement dans ledit trou axial (163) réalisé dans ladite tige (155) et une deuxième section (166) est positionnée coaxialement dans ladite deuxième chambre (162), ladite première section (165) et ladite deuxième section (166) étant séparées l'une de l'autre par une contre-bride (167) coaxiale à ladite tige coulissante (164) et contenue à l'intérieur de ladite deuxième chambre (162).

23. Fixateur externe (150) selon la revendication 22), **caractérisé en ce que** ledit groupe élastique (156) comprend également un ressort hélicoïdal (168) contenu à l'intérieur de ladite deuxième chambre (162), autour de ladite deuxième section (166) de ladite tige coulissante (164) et coopérant par contact avec ladite contre-bride (167) et ladite extrémité (157).

24. Fixateur externe (150) selon la revendication 22), **caractérisé en ce que** ladite première section (165) de ladite tige coulissante (164) présente une extrémité arrondie (169) qui coopère par contact avec lesdits moyens de réglage (158).

25. Fixateur externe (150) selon la revendication 24), **caractérisé en ce que** lesdits moyens de réglage (158) comprennent une vis de réglage (171) logée dans un trou fileté (172) réalisé dans ladite tête galbée (159) et dotée d'une tête de manoeuvre (173a) et d'un corps conique (173) se trouvant en contact avec ladite extrémité arrondie (119) de ladite tige coulissante (164).

26. Fixateur externe (150) selon la revendication 22), **caractérisé en ce que** ladite première section (165) de ladite tige coulissante (164) présente une fente passante (176) contenant un pivot de référence (177) disposé transversalement dans ledit élément tubulaire (153).

27. Fixateur externe (150) selon la revendication 20), **caractérisé en ce que** ledit élément tubulaire (153) présente une première partie (178) s'accouplant partiellement dans ledit manchon (154) et une deuxième partie (179) dotée d'un filet externe (180), les deux parties étant séparées l'une de l'autre par une bride annulaire (181).

28. Fixateur externe (150) selon la revendication 27), **caractérisé en ce qu'**une bague fileté (182) est accouplée audit filet externe (180) sur ladite deuxième partie (179) dudit élément tubulaire (153).
